# EUROPEAN PATENT APPLICATION

(11) **EP 2 048 135 A1**
(43) Date of publication of application: **15.04.2009**
(21) Application number: 08163175.6
(22) Date of filing: 11.11.2002
(51) Int. Cl.: C07D 223/28, A61K 31/55, A61P 25/00

(54) **Use of monohydroxycarbamazepine in the treatment of neuropathic pain and related disorders**

(30) Priority: 12.11.2001 GB 0127177; 12.11.2001 GB 0127176; 12.11.2001 GB 0127178
(62) Divisional of application: 02783080.1
(71) Applicant: NOVARTIS AG, 4056 Basel (CH); Novartis Pharma GmbH, 1235 Vienna (AT)
(72) Inventor: Schmutz, Markus, CH-4124, Schoenenbuch (CH)
(74) Representative: Harrison Goddard Foote

(57) **Abstract**

The invention relates to the use of monohydroxycarbamazepine for the treatment of neuropathic pain and related disorders.

## Description

The present invention relates to new pharmaceutical uses of a carbamazepine derivative.

More particularly the present invention relates to new pharmaceutical uses for monohydroxycarbamazepine of formula I

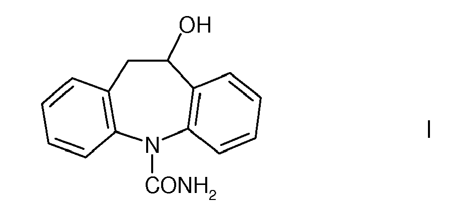

Monohydroxycarbamazepine (10-hydroxy-10,11-dihydro-carbamazepine), the main metabolite of the antiepileptic oxcarbazepine (Trileptal ^{®}) is well known from the literature [see for example Schuetz H. et al., Xenobiotica (GB), 16(8), 769-778 (1986)] and can be prepared synthetically starting from oxcarbazepine according to conventional methods. Monohydroxycarbamazepine has been first disclosed in GB 1310120. The compound is indicated to be suitable for the treatment of psychosomatic disturbances, epilepsy, trigeminal neuralgia and cerebral spasticity.

In accordance with the present invention, it has now surprisingly been found that the compound of formula I is useful in the treatment of affective and attention disorders including e.g. depression and bipolar mood disorders; as well as neuropathic pain and related disorders.

The activity of the compound of formula I in the treatment of affective and attention disorders and of neuropathic pain is evidenced, for example, by its ability to inhibit gamma-aminobutyric acid (GABA) turnover. This is due to feedback inhibition caused by the activating effect of these compounds on GABA transmission.

The role of GABA in bipolar and other mood disorders is unquestioned and topic of several reviews (e.g., Emrich et al. Effect of sodium valproate on mania. The GABA-hypothesis of affective disorders. Arch. Psychiatr. Nervenkr. 1980; 229:1-16; Petty. GABA and mood disorders: a brief review and hypothesis. J. Affect. Disord. 1995; 34:275-81). Drugs effective in (bipolar) mood disorders and also in anxiety and depression, e.g. lithium, valproate and diazepam are known to inhibit the GABA turnover rate. This is due to feedback inhibition caused by the activating effect of these compounds on GABA transmission.

Also, the role of GABA in chronic pain is unquestioned and topic of several reviews (e.g., Stamford. Descending control of pain. Br. J. Anaesth. 1995; 75:217-21). Drugs effective in chronic pain such as valproate are known to inhibit the GABA turnover rate. This is due to feedback inhibition caused by the activating effect of these compounds on GABA transmission.

The activity of the compound of formula I on GABA turnover is evidenced in the following experiment:
The determination of GABA turnover is based on the linear increase in GABA level observed after the maximal inhibition of gamma- aminobutyric acid transaminase (GABA-T). The values obtained with this approach for the rate of GABA synthesis are independent of the inhibitors used and within the catalytic capacity of the enzymes involved in the GABA shunt.

Under these conditions, the compound of formula I dose-dependently inhibits the GABA turnover rate at doses of 30 to about 300 mg/kg p.o.

The activity of the compound of formula I in the treatment of affective and attention disorders treatment is also evidenced, for example, in tests suitable for detecting drugs having potential behavioural desinhibitory and/or sociotropic effects which are thought to be relevant for recovery from social withdrawal, a cardinal feature of depression and related psychiatric conditions. For instance, drug effects on social withdrawal of intruder mice can be evaluated by using the basic method as described in Triangle, 1982, 21:95-105 and J. Clin. Psychiatry, 1994, 55:9 (suppl. B) 4-7.

Within the dose range of 1 to about 100 mg/kg p.o., the compound of formula I increases social investigation in the treated mouse under such experimental conditions.

In view of its anxiolytic-/antidepressant- like stimulating effect on GABA transmission and sociotropic activity, the compound of formula I is useful in the treatment of affective disorders including depression and bipolar disorders, e.g. manic-depressive psychoses, extreme psychotic states e.g. mania, schizophrenia, and excessive mood swings where behavioural stabilization is desired. In addition, the compound is indicated in ADHD (attention deficit hyperactivity disorders) and other attention disorders, e.g. autism, (and) in anxiety states, generalized anxiety and agoraphobia, as well as those behavioural states characterized by social withdrawal e.g. negative symptoms.

The direct activity of the compound of formula I in the treatment of neuropathic pain is evidenced, for example, in the following model of neuropathic pain in the guinea pig:
Dunkin Hartley guinea pigs are anaesthetised with enflurane (in N₂O:O₂ for guinea pigs) and the left sciatic nerve is exposed and partially ligated with thread. This procedure produces a mechanical hyperalgesia, which develops within 2-3 days and is maintained for at least 4 weeks. Paw withdrawal thresholds to a pressure stimulus are measured using an analgesymeter. Mechanical thresholds are taken on both the ipsilateral (ligated) and contralateral (unligated) paw prior to and then up to 6 hours following drug or vehicule administration. Reversal of hyperalgesia at each time point is calculated. Groups of 6 animals are used. Statistical analysis is carried out on withdrawal threshold readings using ANOVA followed by Tukey's HSD test.

In this model, the compound of formula I significantly and dose-dependently reverses neuropathic mechanical hyperalgesia at doses of 10 to about 100 mg/kg p.o. and 3 to about 100 mg/kg s.c.

The activity of the compound of formula I in the treatment of neuropathic pain and related disorders can be confirmed in clinical trials evaluating the efficacy of a compound in treating chronic pain in patients with diabetic neuropathy.

In such studies, the compound of formula I is found to decrease pain severity ratings relative to placebo during the maintenance and follow-up periods, in a statistically significant way.

The compound of formula I is therefore useful in the treatment of neuropathic pain and associated hyperalgesia, including trigeminal and herpetic neuralgia, diabetic neuropathic pain, migraine, causalgia and deafferentation syndromes such as brachial plexus avulsion, and in spasticity and related disorders.

For the above-mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.05 to about 150, preferably from about 0.1 to about 100 mg/kg of animal body weight. In larger mammals, for example humans, an indicated daily dosage is in the range from about 0.5 to about 5000, preferably from about 1 to about 500mg of an agent of the invention, conveniently administered, for example, in divided doses up to four times a day or in sustained release form, for example once a day.

The agent of the invention may be administered by any conventional route, in particular enterally, preferably orally, for example in the form of tablets or capsules, or parenterally, for example in the form of injectable solutions or suspensions.

The agents of the invention can be administered in vivo either alone or in combination with other pharmaceutical agents, e.g. agents effective in the treatment of diseases and conditions in which the human VR1 activation plays a role or is implicated including cyclooxygenase-2 (COX-2) inhibitors, such as specific COX-2 inhibitors (e.g. celecoxib, COX189, and rofecoxib) or in general nonsteroidal anti-inflammatory drugs (NSAIDs) (e.g. acetylsalicylic acid, propionic acid derivatives), tricyclic antidepressants (e.g. Anafranil^{®}, Asendin^{®}, Aventyl^{®}, Elavil^{®}, Endep^{®}, Norfranil^{®}, Norpramin^{®}, Pamelor^{®}, Sinequan^{®}, Surmontil^{®}, Tipramine^{®}, Tofranil^{®}, Vivactil^{®}, Tofranil-PM^{®}), anticonvulsants (e.g. gabapentin), GABA_{B} agonists (e.g. L-baclofen), opioids, vanniloid receptor antagonists and Cannabinoid (CB) receptor agonists, e.g. CB₁ receptor agonists.

The pharmaceutical compositions for separate administration of the combination partners and for the administration in a fixed combination, i.e. a single galenical composition comprising at least two combination partners, according to the invention can be prepared in a manner known per se and are thus suitable for enteral, such as oral or rectal, and parenteral administration to mammals, including man, comprising a therapeutically effective amount of at least one pharmacologically active combination partner alone or in combination with one or more pharmaceutically acceptable carriers, especially suitable for enteral or parenteral application.

The invention further provides the use of the compound of formula I for the manufacture of a pharmaceutical composition for the treatment of affective and attention disorders, neuropathic pain and neuropathic pain related disorders.

The invention furthermore provides a method for the treatment of affective and attention disorders, neuropathic pain and neuropathic pain related disorders in a subject in need of such treatment, which comprises administering to said subject a therapeutically effective amount of the compound of formula 1.

## Claims

1. The use of a compound of the formula 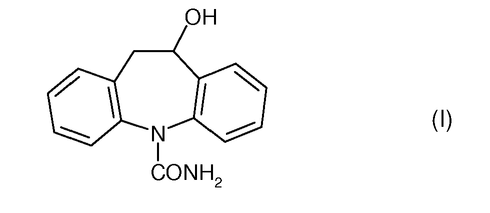 for the manufacture of a medicament for the treatment of a disorder selected from hyperalgesia associated with neuropathic pain, herpetic neuralgia, diabetic neuropathic pain, migraine, causalgia and deafferentation syndromes.

2. The use according to claim 1, wherein the disorder is hyperalgesia associated with neuropathic pain.

3. The use according to claim 1, wherein the disorder is herpetic neuralgia.

4. The use according to claim 1, wherein the disorder is diabetic neuropathic pain.

5. The use according to claim 1, wherein the disorder is migraine.

6. The use according to claim 1, wherein the disorder is causalgia.

7. The use according to claim 1, wherein the disorder is selected from deafferentation syndromes.

8. The use according to claim 7, wherein the disorder is brachial plexus avulsion.

9. The use according to claim 1, wherein the medicament is a combination comprising at least one other pharmacologically active compound in addition to a compound of the formula I.

10. The use according to claim 9, wherein the combination is a fixed combination.

11. A process for the manufacture of a medicament for the treatment of a disorder selected from hyperalgesia associated with neuropathic pain, herpetic neuralgia, diabetic neuropathic pain, migraine, causalgia and deafferentation syndromes, wherein a compound of the formula 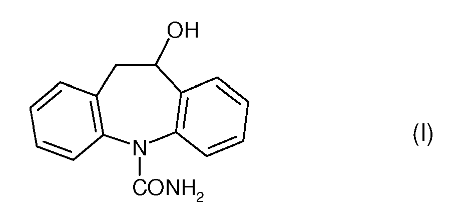 is used as a pharmacologically active compound in association with at least one pharmaceutically acceptable carrier or diluent.

12. A process according to claim 11, wherein the disorder is as defined in any of claims 2 to 8.

13. A process according to claim 11, wherein the medicament is a combination as defined in claim 9 or claim 10.

14. A compound of the formula 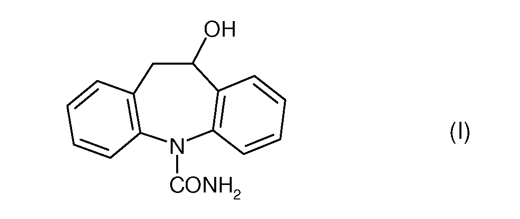 for use in the treatment of a disorder selected from hyperalgesia associated with neuropathic pain, herpetic neuralgia, diabetic neuropathic pain, migraine, causalgia and deafferentation syndromes.

15. A compound according to claim 14, wherein the disorder is as defined in any of claims 2 to 8.
